# EUROPEAN PATENT APPLICATION

(11) **EP 2 369 357 A2**
(43) Date of publication of application: **28.09.2011**
(21) Application number: 11159247.3
(22) Date of filing: 22.03.2011
(51) Int. Cl.: G01R 33/28, A61B 5/055, A61N 1/37

(54) **Method, system and apparatus for monitoring patients**

(30) Priority: 25.03.2010 US 731581
(71) Applicant: General Electric Company, Schenectady, NY 12345 (US)
(72) Inventor: Rantala, Borje Tor, 00670, Helsinki (FI)
(74) Representative: Illingworth-Law, William Illingworth

(57) **Abstract**

A method, system and apparatus are disclosed for monitoring a subject with a transport monitor (11) comprising a measurement unit (31) including at least one magnetic component unit operative when monitoring the subject in absence of a high magnetic field. To maintain the monitoring capacity of a standard transport monitor even in the high magnetic field, the transport monitor is provided with a reconfiguration unit (33) adapted to reconfigure the measurement unit (31) for the high magnetic field so that at least part of the monitoring capability that the measurement unit (31) has in absence of the high magnetic field is preserved in presence of the high magnetic field.

## Description

### BACKGROUND OF THE INVENTION

This disclosure relates to a method and system where an individual is monitored with a transport monitor. The transport monitor here refers to any patient monitoring unit that may be used to monitor a patient during in-hospital transport or when the patient is moved between hospitals. This disclosure also relates to the actual transport monitor and an auxiliary unit for the monitor.

Monitoring the state of a hospital patient requires sophisticated monitoring devices and a rather complex measurement set-up with a plurality of sensing elements being attached to the patient. To provide uninterrupted monitoring even during transportation, a hospital patient may be connected to a so-called transport monitor attached to the patient's bed. Transport monitors allow patients to be moved through the hospital from one care area to another or between hospitals without losing any monitoring information from the patient during the transportation. When the patient is not in transportation, the transport monitor may be connected to a central patient monitor located in an area where the nursing staff may readily follow the state of the patient from the screen thereof. The connection between the two monitors may be implemented through a dedicated cable or through a hospital network, but the connection may also be a wireless point-to-point connection. When the patient is about to be moved to a new care area, the transport monitor is disconnected from the central patient monitor and during the transportation the transport monitor monitors the patient as a standalone monitor. In the new care area, the transport monitor may again be connected to a central patient monitor of that area or reconnected to the hospital network.

The concept of using transport monitors together with central patient monitors is popular and widespread since the total amount of cabling needed may be reduced. This is because the cables connecting the plurality of sensing elements to the actual measurement equipment remain as short as possible, while a single cable is enough to connect the transport monitor to the central monitor. Transport monitors may be provided with different properties and/or capabilities. For example, a transport monitor may be provided with a display unit to display diverse information to the user, while another transport monitor may only collect patient data without displaying the results or even without processing the collected raw data. That is, the monitoring results may be displayed on the screen of the central patient monitor only.

A drawback related to standard transport monitors is that certain hospital care areas, especially MRI (Magnetic Resonance Imaging) rooms, require special equipment and are therefore off limits for standard transport monitors. The MRI environment requires specially designed and shielded components to withstand the high magnetic field involved, and therefore standard transport monitors cannot operate near MRI devices. Furthermore, standard transport monitors tend to emit RF interference that may cause artifact to be present on the MRI image. MRI compatible monitors should also contain minimum amount of ferrous metal, thereby to minimize the attraction force generated by the magnetic field.

Consequently, MRI compatible patient monitors are at present specially designed devices that are, due to the required special properties and the resulting high cost, used in MRI environment only. If a patient is taken to MRI imaging, a standard transport monitor does not provide uninterrupted monitoring but needs to be replaced by an MRI compatible monitor if the patient is to be monitored during the MRI process. However, changing the monitor is cumbersome and requires an excessive amount of time, especially in view of the rather quick MRI imaging process.

### BRIEF DESCRIPTION OF THE INVENTION

The above-mentioned problems are addressed herein which will be comprehended from the following specification. In order to maintain the monitoring capacity of a standard transport monitor even in the high magnetic field of an MRI device, the monitor may be reconfigured so that the magnetic component units that would cause undesired effects in the high magnetic field are made inoperative. Furthermore, the disabled magnetic component units may be replaced by at least one replacement unit that allows the monitoring function related to the disabled magnetic component units to be continued in the MRI environment.

In an embodiment, a method for monitoring a subject comprises monitoring a subject with a transport monitor comprising a measurement unit provided with at least one magnetic component unit, the measurement unit being configured to use the at least one magnetic component unit when monitoring the subject in absence of a high magnetic field. The method further comprises reconfiguring the transport monitor for monitoring the subject in presence of the high magnetic field, wherein the reconfiguring preserves at least part of the monitoring capability that the measurement unit has in absence of the high magnetic field.

In further embodiments, a system or apparatus for monitoring a subject comprises a transport monitor comprising (a) a measurement unit configured to monitor a subject, the measurement unit comprising at least one magnetic component unit operative when monitoring the subject in absence of a high magnetic field and (b) a reconfiguration unit adapted to reconfigure the transport monitor for the high magnetic field so that at least part of the monitoring capability that the measurement unit has in absence of the high magnetic field is preserved in presence of the high magnetic field.

In a still further embodiment, an auxiliary unit for a transport monitor intended for monitoring a subject comprises a first interface for receiving control commands from a transport monitor external to the auxiliary unit, a pneumatic pump and valve unit comprising at least one pump and at least one valve, the pump and valve unit being responsive to the control commands received through the first interface, and a second interface configured to connect the pump and valve unit pneumatically to a blood pressure measurement unit of the external transport monitor.

Various other features, objects, and advantages of the invention will be made apparent to those skilled in the art from the following detailed description and accompanying drawings.

### BRIEF DESCRIPTION OF THE DRAWINGS

FIG. 1 is a schematic diagram illustrating one embodiment of a system for monitoring a subject;
FIG. 2 is a flow diagram illustrating an example of the selection of the operation mode in the transport monitor;
FIG. 3 illustrates the basic components of a transport monitor operative in a high magnetic field;
FIG. 4 illustrates NIBP measurement in the monitor of FIG. 3;
FIG. 5 illustrates one embodiment of the NIBP unit of the monitor of FIG. 4;
FIG. 6 illustrates another embodiment of the NIBP unit of the monitor of FIG. 4; and
FIG. 7 illustrates an example of the disablement of the magnetic component units in a power supply chain of a transport monitor.

### DETAILED DESCRIPTION OF THE INVENTION

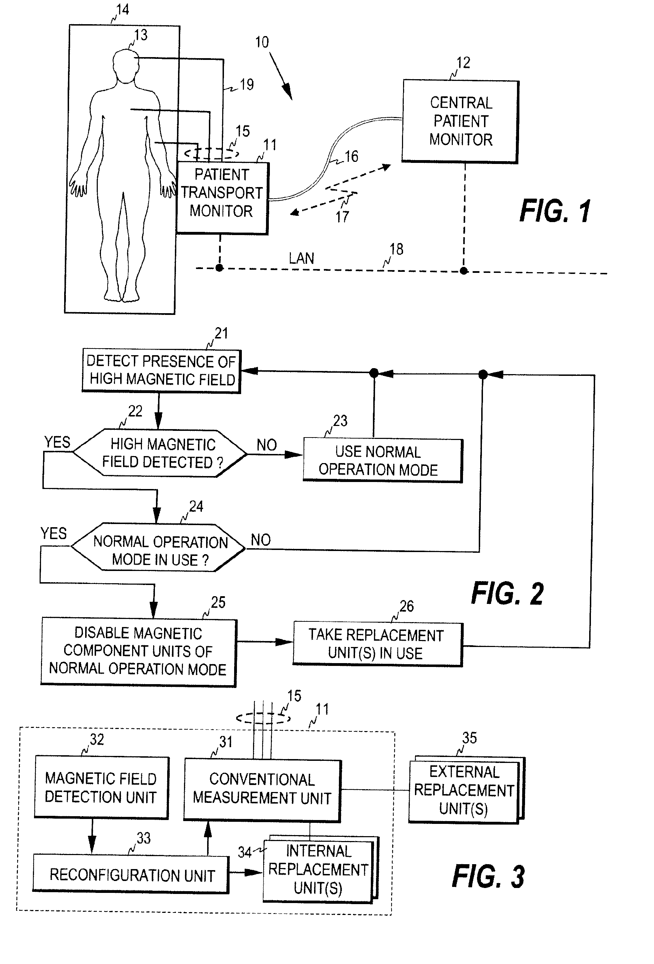

Referring to FIG. 1, a patient monitoring system 10 comprises a transport monitor 11, located typically at bedside, and a central patient monitor 12 located further away from the subject 13 to be monitored, at a location where the nursing staff may supervise the state of the subject from the screen of the central patient monitor. The transport monitor collects physiological data from the subject and transmits the data to the central patient monitor.

The transport monitor is similar to a standard transport monitor and operates like a standard transport monitor. However, this applies to the normal operation of the transport monitor 11 only. In other words, in normal operation the transport monitor is not compatible with the harsh MRI environment and cannot be used near an MRI device. To make the transport monitor MRI compatible, it is provided with a special operation mode in which it is capable of operating in the MRI environment even though it is not provided with the shielding and other special properties of a conventional MRI-compatible monitor. This is discussed below in connection with FIG. 2.

The transport monitor normally acquires a plurality of physiological signals 15 from the subject, where one physiological signal corresponds to one measurement channel. The physiological signals typically comprise several types of signals, such as ECG, EEG, blood pressure, and plethysmographic signals. Based on the raw real-time physiological signal data obtained from the subject, a plurality of physiological parameters may be determined. A physiological parameter here refers to a variable calculated from the waveform data of one or more of the physiological signals acquired from the subject. If a physiological parameter is derived from more than one physiological signal, i.e. from more than one measurement channel, the said physiological signals are usually of the same signal type. The physiological parameter may thus also represent a waveform signal value determined over a predefined period of time, although the physiological parameter is typically a distinct parameter derived from one or more measurement channels, such as heart rate derived from an ECG signal or an SPO2 value derived from a plethysmographic signal. Each signal parameter may be assigned one or more alarm limits to alert the nursing staff when the parameter reaches or crosses the alarm limit.

The transport monitor may be connected to the central patient monitor in several ways, depending on the location of the central patient monitor 12, for example. If the two monitors are close enough to each other, they may be connected to each other through a dedicated cable 16, for example. However, the connection 17 between the two terminals may also be a wireless one, or implemented through hospital network 18. The transport monitor typically determines the physiological parameters from the subject and transmits the parameters to the central patient monitor. However, it also possible that the central patient monitor determines new physiological parameters based on the data collected by the transport monitor. In an extreme case, the transport monitor only collects and transmits the raw data to the central patient monitor. Consequently, the capability level of the transport monitor may vary and thus also the sharing of tasks between the transport monitor and the central patient monitor. At any rate, an important advantage of all transport monitors is that they reduce the total amount of cabling needed, since the sensor cables 19 remain short.

During transportation of the subject through the hospital the transport monitor operates as a standalone monitor collecting physiological data from the subject. The data collected from the subject during the transportation may be transmitted to the new central patient monitor, to which the transport monitor is connected in the new care area.

As discussed above, the transport monitor 11 is provided with a special operation mode to make it MRI-compatible. FIG. 2 illustrates an example of the selection of the operation mode in the transport monitor. The operation mode of the transport monitor depends on whether a high magnetic field is present or not, i.e. whether or not the monitor is near an MRI device (or another device producing a high magnetic field). The detection of the magnetic field may be automatic. That is, the transport monitor may continuously sense whether a high magnetic field is present (step 21). When a high magnetic field is not detected, the transport monitor confines to its normal operation mode in which it operates as a standard transport monitor (steps 22 and 23). However, if the transport monitor detects a high magnetic field during normal operation mode (steps 22 and 24/yes), the monitor enters an MRI operation mode by disabling the magnetic component units used in normal operation mode (step 25). The magnetic component unit here refers to a unit whose operation relies on magnetic properties. Such units typically include components that contain ferromagnetic materials. In the high magnetic field of the MRI device such materials are saturated and if the unit is not damaged, at least a malfunction is caused by the high external magnetic field produced by the MRI device. In a standard transport monitor, such components are typically used in blood pressure measurement, for example. In addition to the disablement of the magnetic component units, the transport monitor may take replacement units into use (step 26). That is, instead of using the internal magnetic component units, the transport monitor may use replacement units in the MRI operation mode.

The transport monitor remains in the MRI operation mode as long as the high magnetic field is detected and returns to the normal operation mode when the high magnetic field is no more present (step 22/no). In the normal operation mode, the magnetic component units are again in use and the transport monitor operates as a standard monitor incompatible with the MRI environment. For the duration of the MRI process the transport monitor may thus be converted, or may convert itself, to an MRI-compatible monitor, while otherwise the monitor operates as a standard transport monitor.

FIG. 3 illustrates the basic units of one embodiment of the transport monitor 11. The core of the monitor is a conventional measurement unit 31 configured to acquire the physiological signal data from the patient. The measurement unit comprises magnetic component units that render the measurement unit inoperative in presence of a high magnetic field. To preserve the monitoring ability of the measurement unit at least partially regardless of the presence of a high magnetic field, the monitor comprises a reconfiguration unit 33 adapted to reconfigure the monitor so that at least partial monitoring ability may be preserved in the high magnetic field. A magnetic field detection unit 32 may notify the reconfiguration unit when a high magnetic field, i.e. a magnetic field whose strength reaches a preset threshold value, is detected. In response to the notification, the reconfiguration unit may reconfigure the monitor by disabling all or selected magnetic component units of the measurement unit and by connecting one or more replacement units 34, 35 to the measurement unit, so that the monitoring may be continued in presence of the high magnetic field. In view of the operation, the reconfiguration unit may thus be divided into two logical units; a disablement unit configured to disable desired magnetic component units of the measurement unit and a commissioning unit that allows the introduction of one or more replacement units operative in the MRI operation mode. The units taken into use in the MRI operation mode may be internal or external replacement unit(s) 34, respectively 35. The reconfiguration unit may also operate in the opposite direction; when the detection unit 32 indicates that the high magnetic field is no more present, the reconfiguration unit may disconnect/disable the external/internal replacement units and re-enable the internal magnetic component units, thereby to restore the normal operation mode. As discussed below, in case of an external replacement unit the measurement unit may include an additional interface for connecting a replacement unit to the measurement unit.

Although the detection of the magnetic field is logically separate from the actual reconfiguration, the magnetic field detection unit 32 may in practice be implemented as an integral part of the reconfiguration unit. However, as automatic detection of the magnetic field is not necessary and as the detection and reconfiguration are logically different operations, the magnetic field detection unit is presented as a distinct unit in FIG. 3.

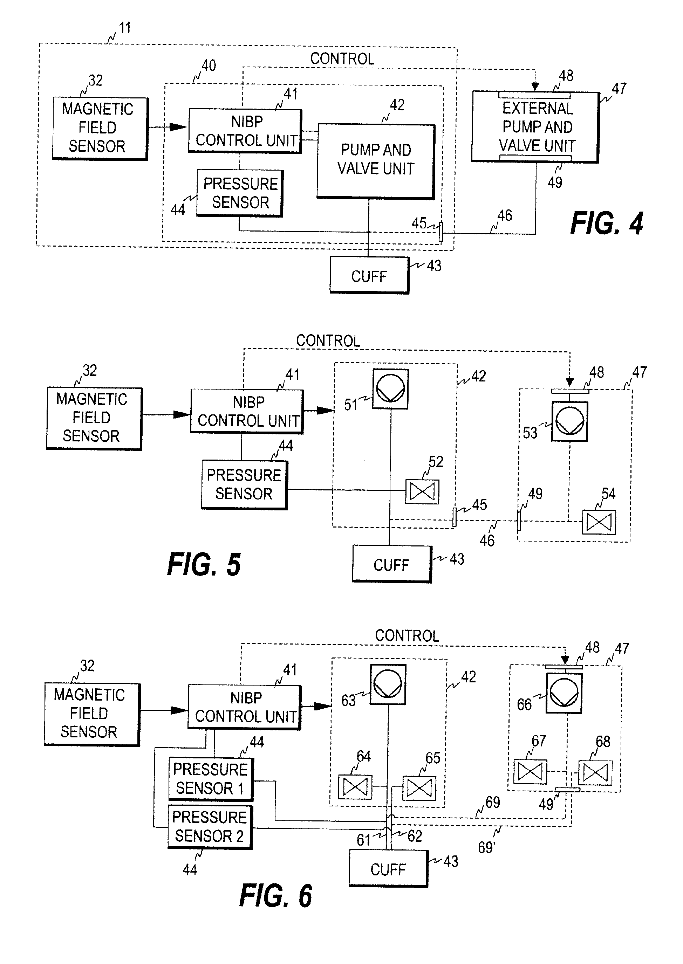

As mentioned above, magnetic component units are typically used in the blood pressure measurement unit of a transport monitor. In a NIBP (non-invasive blood pressure) unit of a transport monitor, a magnetic component unit is typically formed by a magnetic pump and one or more magnetic valves. FIG. 4 illustrates an example of the units responsible for the NIBP measurement in the transport monitor 11. The transport monitor 11 comprises a conventional NIBP unit 40 that is responsible for the NIBP measurement in the normal operation mode of the monitor. The NIBP unit comprises a control unit 41 that controls a (pneumatic) pump and valve unit 42 of the NIBP unit. The pump and valve unit is connected to the cuff 43 attached to the subject. A pressure sensor 44 measures the pressure of the cuff and supplies the measurement results to the control unit 41. In a standard NIBP unit, the pump and valve unit normally contains a magnetic pump and magnetic valves that cannot be used if the transport monitor is in the high magnetic field of an MRI device.

When the transport monitor is in normal operation mode, the operation of the NIBP unit 40 corresponds to that of a conventional NIBP unit. In other words, the control unit controls the pump and valve unit to inflate and deflate the cuff and determines the blood pressure based on the pressure readings obtained from the pressure sensor.

For the MRI operation mode, the NIBP unit 40 is provided with an auxiliary interface 45 to which a hose 46 of an external pump and valve unit 47 may be connected. The external pump and valve unit may be located at a fixed location within a short distance from the MRI device, but yet far enough so that the magnetic field remains low at the said location. In practice, the distance between the MRI device and the external pump and valve unit 47 may be 3 to 5 meters, for example, to ensure that the magnetic field is low enough at the external unit. The external unit may also be outside the MRI room.

When the transport monitor 11 is brought close to the MRI device, a magnetic field sensor 32 of the monitor detects the high magnetic field. As a result, the NIBP unit enters the MRI operation mode by disabling the internal pump and valve unit 42. The user of the monitor may then connect the hose 46 of the external pump and valve unit to the NIBP unit. When the connection is ready, the control unit controls the external pump and the valve unit in a similar way as it controls the internal pump and valve unit 42 in the normal operation mode. The control channel between the control unit and the external pump and valve unit may be wireless. The external pump and valve unit is thus provided with a control interface 48 configured to receive control commands from the NIBP control units of the transport monitors visiting the MRI environment, and with a pneumatic interface 49 for connecting the unit pneumatically to the auxiliary interface of the NIBP unit of such a visiting transport monitor.

Thus, the magnetic field sensor and the control unit here form a reconfiguration unit that is adapted to reconfigure the monitor so that it may preserve its monitoring capability in the MRI environment. The monitor further includes a pneumatic interface 45 for connecting the replacement system, i.e. the external pump and valve unit, which replaces the disabled magnetic component unit, i.e. the internal pump and valve unit, in the MRI operation mode. Since the external pump and valve unit is further away from the MRI device and therefore in low magnetic field, it may contain magnetic components similarly as the internal pump and valve unit does.

FIG. 5 illustrates an example of the internal and external pump and valve units 42, 47, each comprising in this embodiment a pump and a bleed valve. In normal operation mode, the control unit commands internal pump 51 to inflate the cuff. In MRI operation mode, the internal unit 42 is disabled and the external pump and valve unit 47 is connected through the auxiliary interface 45 to the cuff hose. The control unit then commands the external pump 53 to inflate the cuff via hose 46. In normal operation mode, the control unit opens internal bleed valve 52 to deflate the cuff, while in the MRI operation mode the control unit opens external bleed valve 54 (internal bleed valve 52 is closed). The pressure sensor 44 measures the pressure of the cuff in both operation modes.

FIG. 6 illustrates another example of the internal and external pump and valve units 42, 47. It is assumed in this example that the cuff is a dual hose cuff. That is, the cuff is inflated via one hose and deflated via the other. In the embodiment of the figure, the cuff is inflated via hose 61 and deflated via hose 62. As in the example of FIG. 5, the internal and external pump and valve units 42, 47 are substantially similar to each other; each unit now comprises a pump and two valves. In normal operation mode, the control unit 41 commands internal pump 63 to inflate the cuff via hose 61. In MRI operation mode, the internal pump and valve unit 42 is disabled and the external pump and valve unit 47 is connected to the cuff via a dual hose connection. The control unit then commands external pump 66 to inflate the cuff via hoses 69 and 61. In normal operation mode, the control unit opens internal bleed valve 65 to deflate the cuff via hose 62, while in the MRI operation mode the control unit opens external bleed valve 68 (internal bleed valve 65 is closed) to deflate the cuff via hoses 62 and 69'. In this embodiment, two pressure sensors 44 are used to measure the pressure from both cuff hoses. Valves 64 and 67 are connected to the inflation channel for safety purposes.

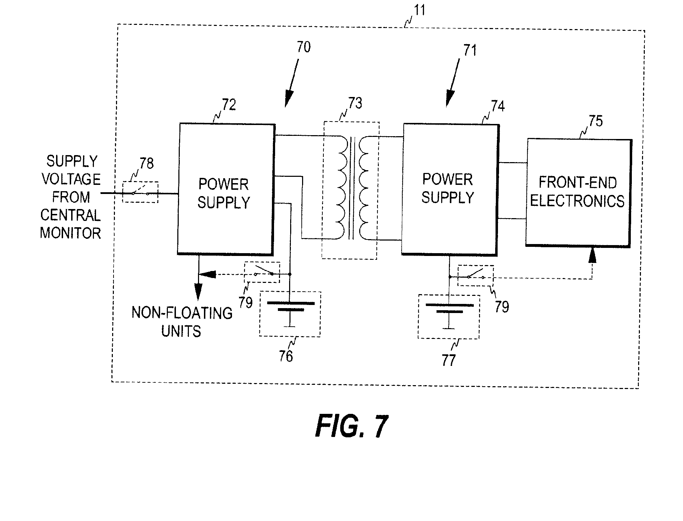

In addition to the NIBP unit, a standard transport monitor typically includes magnetic components in an isolation transformer used to achieve a so-called patient isolation in the power supply chain of the monitor. FIG. 7 illustrates the power supply chain of a typical transport monitor. The isolation transformer 73 divides the monitor into two sections: a base section 70 and a floating section 71. The base section may also be called a non-floating section. The isolation transformer galvanically separates the patient ground potential, i.e. floating or non-earth ground, from the ground potential of the base section. It is assumed in the example of FIG. 7 that the central patient monitor provides a supply voltage to the transport monitor when the transport monitor is connected to the central patient monitor. The supply voltage is supplied to a DC/DC power supply 72 of the base section. When the transport monitor receives the supply voltage, power supply 72 produces supply voltage for the units of the base section, such as the NIBP unit. A power supply 74 of the floating section in turn receives a supply voltage through the isolation transformer and produces power to the front-end electronics 75 of the measurement unit 31. In the example of FIG. 7, both sections of the monitor are provided with a chargeable battery. In normal operation mode, i.e. when the monitor receives a supply voltage from an external power source, such as the central patient monitor, power supply 72 charges battery 76, while power supply 74 charges battery 77. When in MRI mode, the connection to the external power source is disconnected. The non-floating battery 76 then supplies power to the units of the base section and the floating battery 77 to the front-end electronics. This is illustrated with switches 78 and 79, in which the position denoted with a continuous line corresponds to normal operation mode and the position denoted with the dashed lines to MRI operation mode.

In the example of FIG. 7, the power supplies and the isolation transformer 73 form the magnetic component unit that is disabled and the batteries the (internal) replacement units that are taken into use in the MRI operation mode. The reconfiguration unit comprises in this case the switches and the associated control mechanism needed to toggle between the power sources. Since the isolation transformer is the magnetic component that needs to be disabled in each case when entering the MRI mode, it is possible that in another embodiment power supply 72 remains operative when entering the MRI operation mode. In this embodiment, battery 76 is thus omitted and power supply 72 is implemented without magnetic components to preserve its ability to operate in the high magnetic field.

The magnetic component units to be disabled may depend on the construction of the transport monitor. Furthermore, the type and amount of the replacing components may depend on whether full monitoring capacity is to be preserved in the MRI environment or whether it is acceptable to skip the recording of some physiological data for the duration of the MRI process. The apparatus may also be provided with selection tools for selecting the monitoring functions that are to be preserved in the MRI imaging process. Consequently, all magnetic component units that are inoperative in MRI environment may be disabled, while all of them are not necessarily replaced by a replacement unit, if it is enough to preserve a limited selection of monitoring capabilities in the MRI operation mode.

Using the above-described mechanism, low cost transport monitors may be used in the MRI environment to provide virtually uninterrupted monitoring of the patient. The changes needed in a conventional transport monitor are rather insignificant, and thus also the costs remain low. More changes are needed in the MRI environment to equip the said environment with the external pump and valve unit 47. However, these changes are not widely needed, since a typical hospital may have only one or two MRI devices.

The degree of automation in the transition to and from the MRI operation mode may vary depending on the implementation. For example, there may be no automatic detection of the high magnetic field, but the user may have to activate the transition manually. If automatic detection of a high magnetic field is used, the predetermined threshold value causing the detection of the presence of the high magnetic field may vary. Depending on the implementation, the amount of manual connections required from the user may also vary. For example, the user may have to establish the control connection between the NIBP unit and the external pump and valve unit, if the said connection is not a wireless one. The monitor may also instruct the user in various ways when the user is about to arrive in or leave from the MRI environment.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural or operational elements that do not differ from the literal language of the claims, or if they have structural or operational elements with insubstantial differences from the literal language of the claims.

Various aspects of the invention are now set out in the following numbered clauses:
1. A method for monitoring a subject, the method comprising:
   - monitoring a subject with a transport monitor comprising a measurement unit provided with at least one magnetic component unit, the measurement unit being configured to use the at least one magnetic component unit when monitoring the subject in absence of a high magnetic field; and
   - reconfiguring the transport monitor for monitoring the subject in presence of the high magnetic field, wherein the reconfiguring preserves at least part of the monitoring capability that the measurement unit has in absence of the high magnetic field.
2. The method according to clause 1, further comprising detecting whether the high magnetic field is present.
3. The method according to clause 1 or clause 2, wherein the reconfiguring comprises disabling the at least one magnetic component unit when the detecting indicates that the high magnetic field is present.
4. The method according to any preceding clause, wherein the reconfiguring further comprises taking, in response to the disabling, at least one replacement unit into use.
5. The method according to any preceding clause, wherein
   - the disabling comprises disabling the at least one magnetic component unit, in which the at least one magnetic component unit is a pump and valve unit of a blood pressure measurement unit contained in the measurement unit; and
   - the taking into use comprises taking the at least one replacement unit into use, in which the at least one replacement unit is an external pump and valve unit located in low magnetic field.
6. The method according to any preceding clause, wherein
   - the disabling comprises disabling the at least one magnetic component unit, in which the at least one magnetic component unit comprises an isolation transformer in a power supply chain of the transport monitor; and
   - the taking into use comprises taking the at least one replacement unit into use, in which the at least one replacement unit comprises at least one chargeable battery within the transport monitor.
7. A system for monitoring a subject, the system comprising a transport monitor comprising (a) a measurement unit configured to monitor a subject, the measurement unit comprising at least one magnetic component unit operative when monitoring the subject in absence of a high magnetic field and (b) a reconfiguration unit adapted to reconfigure the transport monitor for the high magnetic field so that at least part of the monitoring capability that the measurement unit has in absence of the high magnetic field is preserved in presence of the high magnetic field.
8. The system according to clause 7, wherein the reconfiguration unit comprises a magnetic field sensor configured to detect whether the high magnetic field is present.
9. The system according to clause 7 or clause 8, wherein the reconfiguration unit is adapted to disable the at least one magnetic component unit when the magnetic field sensor indicates that the high magnetic field is present.
10. The system according to any of clauses 7 to 9, further comprising at least one replacement unit, wherein the reconfiguration unit is adapted to take, in response to disablement of the at least one magnetic component unit, the at least one replacement unit into use.
11. The system according to any of clauses 7 to 10, wherein
   - the at least one magnetic component unit comprises a pump and valve unit of a blood pressure measurement unit contained in the measurement unit; and
   - the at least one replacement unit is an external pump and valve unit located in low magnetic field.
12. The system according to any of clauses 7 to 11, wherein the external pump and valve unit comprises magnetic components.
13. The system according to any of clauses 7 to 12, wherein
   - the at least one magnetic component unit comprises an isolation transformer in a power supply chain of the transport monitor; and
   - the at least one replacement unit comprises at least one chargeable battery within the transport monitor.
14. An apparatus for monitoring a patient, the apparatus comprising
   - a measurement unit configured to monitor a subject, the measurement unit comprising at least one magnetic component unit operative when monitoring the subject in absence of a high magnetic field; and
   - a reconfiguration unit adapted to reconfigure the apparatus for the high magnetic field so that at least part of the monitoring capability that the measurement unit has in absence of the high magnetic field is preserved in presence of the high magnetic field.
15. The apparatus according to clause 14, wherein the reconfiguration unit comprises a magnetic field sensor configured to detect whether the high magnetic field is present.
16. The apparatus according to clause 14 or clause 15, wherein the reconfiguration unit is adapted to disable the at least one magnetic component unit when the magnetic field sensor indicates that the high magnetic field is present.
17. The apparatus according to any of clauses 14 to 16, further comprising at least one replacement unit, wherein the reconfiguration unit is adapted to take, in response to disablement of the at least one magnetic component unit, the at least one replacement unit into use.
18. The apparatus according to any of clauses 14 to 17, wherein
   - the at least one magnetic component unit comprises a pump and valve unit of a blood pressure measurement unit contained in the measurement unit; and
   - the apparatus comprises an interface unit allowing connection of at least one replacement unit to the apparatus, thereby to replace the pump and valve unit by the at least one replacement unit.
19. The apparatus according to any of clauses 14 to 18, wherein
   - the at least one magnetic component unit comprises an isolation transformer in a power supply chain of the apparatus; and
   - the at least one replacement unit comprises at least one chargeable battery within the apparatus.
20. An auxiliary unit for a transport monitor intended for monitoring a subject, the auxiliary unit comprising:
   - a first interface for receiving control commands from a transport monitor external to the auxiliary unit;
   - a pneumatic pump and valve unit comprising at least one pump and at least one valve, the pump and valve unit being responsive to the control commands received through the first interface; and
   - a second interface configured to connect the pump and valve unit pneumatically to a blood pressure measurement unit of the external transport monitor.

## Claims

1. A method for monitoring a subject, the method comprising:
- monitoring a subject (13) with a transport monitor (11) comprising a measurement unit (31) provided with at least one magnetic component unit (42; 73), the measurement unit being configured to use the at least one magnetic component unit when monitoring the subject in absence of a high magnetic field; and
- reconfiguring (25, 26) the transport monitor (11) for monitoring the subject in presence of the high magnetic field, wherein the reconfiguring preserves at least part of the monitoring capability that the measurement unit has in absence of the high magnetic field.

2. The method according to claim 1, further comprising detecting (21) whether the high magnetic field is present.

3. The method according to claim 1 or claim 2, wherein the reconfiguring comprises disabling (25) the at least one magnetic component unit (42; 73) when the detecting indicates that the high magnetic field is present.

4. The method according to any preceding claim, wherein the reconfiguring further comprises taking (26), in response to the disabling, at least one replacement unit (34, 35; 47; 77) into use.

5. The method according to any preceding claim, wherein
- the disabling comprises disabling the at least one magnetic component unit, in which the at least one magnetic component unit is a pump and valve unit (42) of a blood pressure measurement unit (40) contained in the measurement unit (31); and
- the taking into use comprises taking the at least one replacement unit into use, in which the at least one replacement unit is an external pump and valve unit (47) located in low magnetic field.

6. The method according to any preceding claim, wherein
- the disabling comprises disabling the at least one magnetic component unit, in which the at least one magnetic component unit comprises an isolation transformer (73) in a power supply chain of the transport monitor; and
- the taking into use comprises taking the at least one replacement unit into use, in which the at least one replacement unit comprises at least one chargeable battery (76, 77) within the transport monitor.

7. An arrangement for monitoring a subject, the arrangement comprising a transport monitor (11) comprising (a) a measurement unit (31) configured to monitor a subject (13), the measurement unit comprising at least one magnetic component unit (42; 73) operative when monitoring the subject in absence of a high magnetic field and (b) a reconfiguration unit (33) adapted to reconfigure the transport monitor for the high magnetic field so that at least part of the monitoring capability that the measurement unit has in absence of the high magnetic field is preserved in presence of the high magnetic field.

8. The arrangement according to claim 7, wherein the reconfiguration unit (33) comprises a magnetic field sensor (32) configured to detect whether the high magnetic field is present.

9. The arrangement according to claim 7 or claim 8, wherein the reconfiguration unit (33) is adapted to disable the at least one magnetic component unit (42; 73) when the magnetic field sensor (32) indicates that the high magnetic field is present.

10. The arrangement according to any of claims 7 to 9, further comprising at least one replacement unit (34, 35; 47; 77), wherein the reconfiguration unit is adapted to take, in response to disablement of the at least one magnetic component unit, the at least one replacement unit into use.

11. The arrangement according to any of claims 7 to 10, wherein
- the at least one magnetic component unit comprises a pump and valve unit (42) of a blood pressure measurement unit (40) contained in the measurement unit (31); and
- the at least one replacement unit is an external pump and valve unit (47) located in low magnetic field.

12. The arrangement according to any of claims 7 to 11, wherein the external pump and valve unit (47) comprises magnetic components.

13. The arrangement according to any of claims 7 to 12, wherein
- the at least one magnetic component unit comprises an isolation transformer (73) in a power supply chain of the transport monitor; and
- the at least one replacement unit (34) comprises at least one chargeable battery (76, 77) within the transport monitor.

14. The arrangement according to any of claims 7 to 13, wherein
- the at least one magnetic component unit comprises a pump and valve unit (42) of a blood pressure measurement unit (40) contained in the measurement unit (31); and
- the transport monitor comprises an interface unit (45) allowing connection of at least one replacement unit (47) to the apparatus, thereby to replace the pump and valve unit by the at least one replacement unit.

15. An auxiliary unit for a transport monitor intended for monitoring a subject, the auxiliary unit comprising:
- a first interface (48) for receiving control commands from a transport monitor (11) external to the auxiliary unit;
- a pneumatic pump and valve unit comprising at least one pump (53; 66) and at least one valve (54; 67, 68), the pump and valve unit being responsive to the control commands received through the first interface (48); and
- a second interface (49) configured to connect the pump and valve unit pneumatically to a blood pressure measurement unit (40) of the external transport monitor (11).
